# EUROPÄISCHE PATENTSCHRIFT

(11) **EP 1 950 281 B1**
(45) Veröffentlichungstag und Bekanntmachung des Hinweises auf die Patenterteilung: **09.09.2009**
(21) Anmeldenummer: 08001489.7
(22) Anmeldetag: 28.01.2008
(51) Int. Cl.: C12C 13/00, C12G 1/00, C12G 3/00, C12M 1/21, G01N 33/00, G01N 33/14

(54) **Messeinrichtung zur Bestimmung der Aktivität eines Fermentationsprozesses**
Measuring device for determining the activity of a fermentation process
Dispositif de mesure destiné à la détermination de l'activité d'un processus de fermentation

(30) Priorität: 29.01.2007 DE 102007005205
(43) Veröffentlichungstag der Anmeldung: 30.07.2008
(73) Patentinhaber: fp sensor systems GmbH, 63927 Bürgstadt (DE)
(72) Erfinder: Petter, Peter, Dr., 63920 Grossheubach (DE); Fürst, Peter, 63927 Bürgstadt (DE)

(56) Entgegenhaltungen:
- WO-A-03/067241
- DE-U1- 20 303 829
- GB-A- 2 391 622
- US-A- 5 476 573

## Beschreibung

Die Erfindung betrifft eine Messeinrichtung zur Bestimmung der Aktivität eines in einem Fermentationsgefäß ablaufenden Fermentationsprozesses nach dem Oberbegriff des Anspruchs 1.

Fermentationsprozesse, beispielsweise die Vergärung von Traubenmost oder anderen Fruchtsäften, werden üblicherweise in entsprechend geeigneten Gefäßen, beispielsweise speziellen Gärtanks, durchgeführt. Bei der Fermentation im Fermentationsgefäß entstehen dabei gasförmige Reaktionsprodukte, beispielsweise Kohlendioxid, die den Verlauf des Fermentationsprozesses charakterisieren. Es sind deshalb Messeinrichtungen bekannt, mit denen das über eine Verbindungsleitung aus dem Fermentationsgefäß abgeführte gasförmige Reaktionsprodukt über einen Gasmesssensor geleitet werden kann, um durch Messung des gasförmigen Reaktionsproduktes auf die Aktivität des Fermentationsprozesses schließen zu können. Eine solche Messeinrichtung ist beispielsweise aus der DE 10 2004 011 413 A1 bekannt.

Praxisbeobachtungen haben gezeigt, dass die bekannten Messeinrichtungen vielfach falsche Messwerte liefern, obwohl die Messeinrichtung ordnungsgemäß installiert und kalibriert ist. Genauere Untersuchungen haben dabei gezeigt, dass die falschen Messwerte der Messeinrichtung auf der Kontamination des Gasmesssensors mit Schaumrückständen beruhen. Denn bei der Fermentation im Fermentationsgefäß kann es zu einem Überschäumen kommen, wobei der überschäumende Schaum dann durch die Verbindungsleitung in die Messeinrichtung eintritt und dort den Gasmesssensor kontaminiert.

Ausgehend von dem in der DE 10 2004 011 413 A1 beschriebenen Stand der Technik ist es deshalb Aufgabe der vorliegenden Erfindung, eine neue Messeinrichtung zur Bestimmung der Aktivität von Fermentationsprozessen vorzuschlagen, die eine höhere Messzuverlässigkeit aufweist.

Diese Aufgabe wird durch eine Messeinrichtung nach der Lehre des Anspruchs 1 gelöst.

Vorteilhafte Ausführungsformen der Erfindung sind Gegenstand der Unteransprüche.

Die erfindungsgemäße Messeinrichtung beruht auf dem Grundgedanken, dass die Messeinrichtung vor dem Eintritt von überschäumendem Schaum geschützt werden muss, um die Kontamination des Gasmesssensors mit Schaumrückständen zu verhindern. Erfindungsgemäß ist es deshalb vorgesehen, vor dem Gasmesssensor eine Schaumschutzeinrichtung anzuordnen, mit der der Durchtritt von im Fermentationsgefäß gebildetem Schaum verhindert werden kann. Im Ergebnis ist dadurch gewährleistet, dass der überschäumende Schaum nicht mehr in die Messeinrichtung eintritt und eine Beschädigung des Gasmesssensors durch Schaumrückstände somit zuverlässig ausgeschlossen ist.

In welcher Art die Schaumschutzeinrichtung ausgebildet ist, ist grundsätzlich beliebig. Nach einer ersten bevorzugten Ausführungsform dient als Schaumschutzeinrichtung eine passiv wirkende Schaumschutzeinrichtung, bei der eine Schaumbarriere vorgesehen ist. Vorteil derartiger passiv wirkender Schaumschutzeinrichtungen ist es, dass diese aufgrund ihrer Passivität nicht ausfallen können und somit beispielsweise auch bei Stromausfall wirksam arbeiten.

Als Schaumbarriere haben sich dabei insbesondere gasdurchlässige Gewebe als geeignet erwiesen. Aufgrund der Gasdurchlässigkeit kann das gasförmige Reaktionsprodukt ungehindert aus dem Fermentationsgefäß durch die Verbindungsleitung in die Messeinrichtung eintreten. Sobald übermäßig Schaum im Fermentationsgefäß gebildet wird und durch die Verbindungsleitung zur Messeinrichtung hin überschäumt, wird das Gewebe der Schaumbarriere von der im Schaum enthaltenen Flüssigkeit benetzt. Diese Flüssigkeit verschließt aufgrund ihrer Oberflächenspannung das Gewebe und verhindert einen weiteren Durchtritt von Gas und Flüssigkeit durch das Gewebe. Ein Durchtritt des Schaums zum Messsensor der Messeinrichtung hin ist somit ausgeschlossen.

Als besonders geeignet zur Herstellung einer Schaumbarriere haben sich Gewebe aus einem hydrophoben Material, beispielsweise aus PET oder PTFE, erwiesen.

Das Gewebe der Schaumbarriere sollte dabei außerdem bevorzugt eine 1:2 Köper-Bindung aufweisen.

Die Maschenweite des Gewebes der Schaumbarriere sollte bevorzugt im Bereich von 10 bis 40 µm, insbesondere ungefähr bei 27 µm, liegen. Die Siebfeinheit des Gewebes der Schaumbarriere sollte im Bereich von 100/cm bis 200/cm, insbesondere bei circa 146/cm, gewählt werden. Der Drahtdurchmesser des Gewebes der Schaumbarriere sollte im Bereich von 30 µm bis 50 µm, insbesondere ungefähr bei 42 µm, liegen. Als offene Fläche des Gewebes der Schaumbarriere sollte ein Grad von 10 % bis 20 %, insbesondere ungefähr 15,5 %, vorgesehen sein.

Das Flächengewicht des Gewebes der Schaumbarriere kann im Bereich von 40 g/m² bis 50 g/m², insbesondere bei ungefähr bei 45 g/m², gewählt werden.

Alternativ bzw. additiv zu einer passiv wirkenden Schaumschutzeinrichtung kann auch eine aktiv wirkende Schaumschutzeinrichtung vorgesehen sein. Diese aktiv wirkende Schaumschutzeinrichtung umfasst einen Schaumsensor zur Detektierung von Schaum und eine Absperreinrichtung zur Absperrung des Schaums. Sobald der Schaumsensor das Vorhandensein von Schaum beispielsweise in der Verbindungsleitung detektiert, wird die Absperreinrichtung aktiviert und dadurch verschlossen, so dass der Schaum nicht in Richtung der Messeinrichtung durchtreten kann.

Ein besonders guter Schutz ergibt sich, wenn die aktiv wirkende Schaumschutzeinrichtung der passiv wirkenden Schaumschutzeinrichtung vorgeordnet ist. Vorteil der aktiv wirkenden Schaumschutzeinrichtung ist es nämlich, dass eine Kontamination empfindlicher Teile durch den überschäumenden Schaum grundsätzlich ausgeschlossen ist. Im Ergebnis wird also die passiv wirkende Schaumschutzeinrichtung, die beispielsweise von einem Gewebe gebildet ist, durch die aktiv wirkende Schaumschutzeinrichtung vor Kontaminationen geschützt. Umgekehrt ist ein ausreichender Schaumschutz auch bei Ausfall der aktiv wirkenden Schaumschutzeinrichtung, beispielsweise bei Stromausfall, durch die nachgeordnete passiv wirkende Schaumschutzeinrichtung gewährleistet.

Die Bauart der Absperreinrichtung in der aktiv wirkenden Schaumschutzeinrichtung ist grundsätzlich beliebig. Diese Absperreinrichtung kann beispielsweise in der Art eines Ventils, insbesondere in der Art eines Membranmagnetventils oder eines motorisch angetriebenen Kugelventils, ausgebildet sein.

Auch die Bauart des Schaumsensors in der aktiv wirkenden Schaumschutzeinrichtung ist grundsätzlich beliebig. Besonders geeignet sind kapazitive, conduktive oder optische Sensoren, um den Schaum beispielsweise in der Verbindungsleitung zu detektieren.

Die Kontaktfläche des Schaumsensors, an der der Schaum bei der Detektion zur Anlage kommen kann, sollte dabei bevorzugt aus schmutz- und wasserabweisendern Material, insbesondere aus PET oder PTFE, hergestellt sein, um eine Beeinträchtigung der Funktion des Schaumsensors beim Überschäumen des Schaums durch anhaftende Schaumrückstände auszuschließen.

Weiterhin ist es besonders vorteilhaft, wenn der Schaumsensor der aktiv wirkenden Schutzeinrichtung in einem Detektionsraum angeordnet ist, wobei der Querschnitt des Detektionsraums so groß ist, dass aufgrund des Abstandes zwischen Schaumsensor und gegenüberliegender Innenwandung des Detektionsraums eine dauerhafte Schaumanhaftung ausgeschlossen werden kann. Auf diese Weise wird verhindert, dass sich längerfristiger Schaum auf der Oberfläche des Schaumsensors anlagern kann.

Durch die erfindungsgemäße Schaumschutzeinrichtung wird der Durchtritt des überschäumenden Schaums verhindert, was zu einem Anstieg des Überdrucks im Fermentationsgefäß bzw. in der Verbindungsleitung führen kann. Um Beschädigungen durch einen übermäßigen Überdruck auszuschließen, kann eine Überdruckschutzeinrichtung eingesetzt werden. Durch die Überdruckschutzeinrichtung wird beim Überschreiten einer bestimmten Überdruckgrenze Gas und/oder Schaum abgelassen, so dass der Überdruck wiederum unter die Überdruckgrenze fällt.

Soweit zum Schaumschutz eine Schaumbarriere eingesetzt wird, sollte die Überdruckgrenze, bei der die Überdruckschutzeinrichtung Gas und/oder Schaum ablässt, kleiner als der Druck gewählt werden, der durch die schaumbenetzte Schaumbarriere abgedichtet werden kann. Auf diese Weise ist gewährleistet, dass der an der Schaumbarriere anstehende Schaum nicht durch einen übermäßigen Überdruck durch die Schaumbarriere durchgedrückt wird.

Die Bauart der Überdruckschutzeinrichtung ist grundsätzlich beliebig. Als besonders einfach und zuverlässig haben sich flüssigkeitsgefüllte Siphons für diese Funktion erwiesen. Diese haben insbesondere den Vorteil, dass auch beim Ablassen von Schaum keine Funktionsbeeinträchtigungen durch Schaumablagerungen zu befürchten sind.

Für die Funktion des Überdruckschutzes bei gleichzeitiger Abdichtung ist es bei Verwendung eines Siphons unbedingt erforderlich, dass dieser ausreichend mit Dichtflüssigkeit gefüllt ist. Um dies entsprechend zu überwachen, kann an dem Siphon eine Füllstandsüberwachungseinrichtung vorgesehen sein. Beispielsweise kann die Füllhöhe des Siphons mit einem Flüssigkeitssensor überwacht werden. Sobald der Füllstand unter eine kritische Marke fällt, gibt die Füllstandsüberwachungseinrichtung ein entsprechend geeignetes Warnsignal ab.

Steigt der Druck im Fermentationsgefäß über die definierte Überdruckgrenze des flüssigkeitsgefüllten Siphons, so wird die Flüssigkeit aus dem Siphon nach außen gedrückt, bis Gas austreten kann. Um Flüssigkeitsverluste auch in diesem Falle zu verhindern, kann am äußeren Ende des Siphons ein Speichervolumen vorgesehen sein, in dem bei Gasdurchtritt durch den Siphon die Siphonflüssigkeit aufgenommen werden kann. Sobald der Überdruck dann wieder unter die Überdruckgrenze fällt, fließt die Siphonflüssigkeit wieder aus dem Speichervolumen in den Siphon hinein und dichtet diesen erneut ohne weiteres automatisch ab.

Das im Fermentationsprozess entstehende gasförmige Reaktionsprodukt ist in der Regel hoch angereichert mit Wasser. Dies führt in der Praxis leicht zur Bildung von Kondenswasser an den Innenwänden des gesamten Gas führenden Systems, insbesondere in der Messeinrichtung. Das Kondenswasser kann insbesondere den Gasmesssensor durch Verstopfungen oder Querschnittsverengungen in seiner Funktion beeinträchtigen. Zum Schutz der Messeinrichtung vor Beschädigungen durch Kondenswasser kann deshalb eine Heizeinrichtung vorgesehen werden, mit der die Messeinrichtung zumindest bereichsweise beheizt werden kann. Aufgrund der Beheizung der Messeinrichtung wird der Niederschlag von Kondenswasser in der Messeinrichtung verhindert.

Alternativ bzw. additiv zur Beheizung der Messeinrichtung kann auch eine Wärmeisolierung an der Messeinrichtung vorgesehen sein, um den Niederschlag von Kondenswasser zu verhindern.

Als Gasmesssensoren haben sich insbesondere Gasdurchflusssensoren, beispielsweise Massendurchflusssensoren, als geeignet erwiesen.

Aufgrund verschiedener Anlagengrößen können verschiedene Messbereiche der erfindungsgemäßen Messeinrichtung notwendig sein, was zu einer entsprechenden Vielfalt von Bauteilvarianten führt. Um die Vielfalt von Bauteilvarianten der Messeinrichtung zu verringern, kann parallel zum Gasmesssensor ein Bypass mit definierter Durchflusskennlinie vorgesehen sein. Durch diesen Bypass strömt dann aufgrund der bekannten Durchflusskennlinie eine in Relation zum Durchfluss durch den Gasmesssensor bekannte Gasmenge, so dass durch Messung des Gasmesssensors auf die gesamte Gasmenge geschlossen werden kann. Hat der Bypass beispielsweise die gleiche Durchflusskennlinie wie der Gasmesssensor selbst, so ist der Messwert des Gasmesssensors einfach zu verdoppeln.

Bei der Weinherstellung sind die Benutzer seit Jahrzehnten gewohnt, den Ausstoß des Gärgases und die Gärintensität akustisch und optisch durch den gluckernden Durchtritt des Gärgases am Gäraufsatz des Gärspundes zu erfassen. Um auch bei Verwendung der erfindungsgemäßen Messeinrichtung dieses Informationsbedürfnis der Benutzer zur Angabe der Gärintensität in Form von für den Menschen wahrnehmbaren Signalen zu befriedigen, kann der Gasmesssensor mit einer optischen und/oder akustischen Ausgabeeinheit verbunden werden. Die Messwerte des Gasmesssensors werden an dieser Ausgabeeinheit in Form von für den Menschen wahrnehmbaren Signalen ausgegeben. Dazu kann an der Ausgabeeinheit beispielsweise eine optische Balkenanzeige oder ein Lautsprecher vorgesehen sein, wobei an dem Lautsprecher synthetisch erzeugte Gluckergeräusche abgegeben werden. Diese Art der Ausgabe des Messergebnisses erhöht die Akzeptanz bei den Benutzern, da diese insbesondere an die Beurteilung des Fermentationsprozesses durch Gluckergeräusche gewöhnt sind. Die Messwerte des Gasmesssensors können außerdem auch an ein übergeordnetes Prozessleit- und/oder Regelsystem weitergeleitet werden.

Die Messeinrichtung selber weist regelmäßig einen Gasaustritt auf, um nach der Messung des Reaktionsgases dieses nach außen abzulassen. Um den Eintritt von Schmutzpartikeln am Gasaustritt zu verhindern, kann an dieser Stelle eine Schmutzbarriere, beispielsweise ein gasdurchlässiges Schmutzfanggewebe und/oder eine Schutzkappe, vorgesehen sein.

Ausführungsformen der erfindungsgemäßen Messeinrichtung sind in den Zeichnungen schematisch dargestellt und werden nachfolgend beispielhaft erläutert.

Es zeigen:
- **Fig. 1**: den schematisiert dargestellten Aufbau einer erfindungsgemäßen Messeinrichtung;
- **Fig. 2**: eine Ausführungsform eines Gasmesssensors mit Bypass.

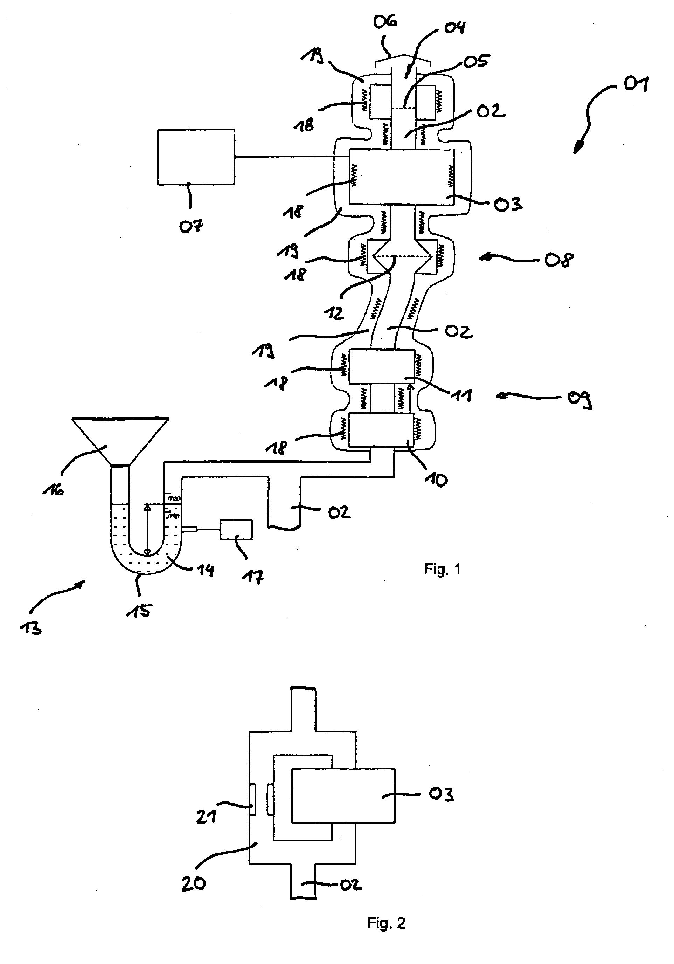

**Fig. 1** zeigt eine Messeinrichtung 01 im schematisierten Querschnitt. Durch eine Verbindungsleitung 02 gelangen gasförmige Reaktionsprodukte aus einem nicht dargestellten Fermentationsgefäß, beispielsweise einem Gärtank, in die Messeinrichtung 01. Die Messeinrichtung 01 ist mit einem Gasmesssensor 03 ausgestattet, in dem die gasförmigen Reaktionsprodukte gemessen werden können, um auf die Aktivität des Fermentationsprozesses zu schließen. Über einen Gasaustritt 04 wird das Fermentationsgas anschließend aus der Messeinrichtung 01 abgelassen. Der Gasaustritt 04 ist dabei durch ein Schmutzfanggewebe 05 und eine Schutzkappe 06 vor dem Eintritt von Schmutzpartikeln geschützt.

Die Messwerte des Gasmesssensors 03 werden an eine Ausgabceinheit 07 weitergeleitet, wo dem Benutzer die Messdaten optisch und/oder akustisch angezeigt werden können, um den Benutzer über die Aktivität des Fermentationsprozesses zu informieren.

Um den Gasmesssensor 03 vor Versschmutzungen durch überschäumenden Schaum aus dem Fermentationsgefäß zu schützen, sind in der Messeinrichtung 01 eine passiv wirkende Schaumschutzeinrichtung 08 und eine aktiv wirkende Schaumschutzeinrichtung 09 vorgesehen. Die aktiv wirkende Schaumschutzeinrichtung 09 besteht aus einem Schaumsensor 10, mit dem die Anwesenheit von Schaum in der Leitung detektiert werden kann, und einer Absperreinrichtung 11. Sobald der Schaumsensor 10 das Vorhandensein von Schaum in der Leitung feststellt, wird die Absperreinrichtung 11 automatisch geschlossen und somit der Durchtritt des Schaums in Richtung des Gasmesssensors 03 verhindert.

Der Schaumschutz in der passiv wirkenden Schaumschutzeinrichtung 08 wird durch eine Schaumbarriere 12 gewährleistet, die in der Art eines Gewebeelements ausgebildet ist. Die passiv wirkende Schaumschutzeinrichtung 08 ist dabei der aktiv wirkenden Schaumschutzeinrichtung 09 nachgeordnet. Im Ergebnis wird somit der Schutz der passiv wirkenden Schaumschutzeinrichtung 08 im Grundsatz durch die aktiv wirkende Schaumschutzeinrichtung 09 gewährleistet. Nur in den Fällen, in denen die aktiv wirkende Schaumschutzeinrichtung 09 ausfällt, beispielsweise bei Stromausfall, kann der Schaum bis zur Schaumbarriere 12 gelangen und wird hier durch das entsprechend vorgesehene Gewebeelement aufgehalten. Allerdings muss das Gewebe nach Rückgang des Schaums dann durch den Benutzer gereinigt werden.

Um Beschädigungen durch Überdruck im Falle der Absperrung des Gasaustritts 04 durch die aktiv wirkende Schaumschutzeinrichtung 09 bzw. die passiv wirkende Schaumschutzeinrichtung 08 zu verhindern, ist eine Überdruckschutzeinrichtung 13 vorgesehen, die in der Art eines mit einer Flüssigkeit 14 gefüllten Siphons 15 ausgebildet ist. Sobald die Schaumschutzeinrichtungen 08 oder 09 einen Gasaustritt verhindern, steigt der Druck in der Verbindungsleitung 02 so weit an, bis die Flüssigkeit 14 entgegen der Schwerkraft so weit in ein Speichervolumen 16 gedrückt ist, dass Gas oder Schaum austreten kann. Der dafür erforderliche Überdruck wird maßgeblich durch die Menge der Flüssigkeit 14 im Siphon 15 bestimmt. Um die Menge in einfacher Weise leicht einfüllen zu können, sind Einfüllmarkierungen für die maximale bzw. die minimale Füllhöhe am Siphon 15 vorgesehen. Außerdem ist am Siphon 15 eine Füllstandsüberwachungseinrichtung 17 vorgesehen, mit der der Füllstand der Flüssigkeit 15 überwacht und bei Unterschreiten gewarnt werden kann.

Um die Bauteile der Messeinrichtung 01 vor Kondenswasser zu schützen, sind in den verschiedenen Bereichen der Messeinrichtung 01 Heizelemente 18 vorgesehen. Außerdem ist die Außenseite der Messeinrichtung 01 mit einer Wärmeisolierung 19 umgeben.

**Fig. 2** zeigt die Möglichkeit zur Parallelanordnung eines Bypasses 20 parallel zum Gasmesssensor 03. Die Gasdurchflusskennlinie im Bypass 20 kann durch Einsätze 21 gezielt eingestellt werden, um im Ergebnis mittels der Messwerte des Gasmesssensors 03 auf den Gesamtgasdurchfluss schließen zu können. Entspricht beispielsweise die Durchflusskennlinie des Bypasses 20 gerade der Durchflusskennlinie des Gasmesssensors 03, so können die Messwerte des Gasmesssensors 03 in einfacher Weise verdoppelt werden, um den tatsächlichen Gasdurchfluss widerzuspiegeln.

### Bezugszeichenliste

- 01: Messeinrichtung
- 02: Verbindungsleitung
- 03: Gasmesssensor
- 04: Gasaustrittsöffnung
- 05: Schmutzfanggewebe
- 06: Schutzkappe
- 07: Ausgabeeinheit
- 08: Passiv wirkende Schaumschutzeinrichtung
- 09: Aktiv wirkende Schaumschutzeinrichtung
- 10: Schaumsensor
- 11: Absperreinrichtung
- 12: Schaumbarriere
- 13: Überdruckschutzeinrichtung
- 14: Flüssigkeit
- 15: Siphon
- 16: Speichervolumen
- 17: Füllstandsüberwachungseinrichtung
- 18: Heizelement
- 19: Wärmeisolierung
- 20: Bypass
- 21: Einsatz

## Patentansprüche

1. Messeinrichtung (01) zur Bestimmung der Aktivität eines in einem Fermentationsgefäß ablaufenden Fermentationsprozesses, insbesondere eines Gärprozesses, wobei die Messeinrichtung (01) mit dem Fermentationsgefäß über eine Verbindungsleitung (02) verbunden werden kann, und wobei gasförmige Reaktionsprodukte aus dem Fermentationsgefäß durch die Verbindungsleitung (02) zu einem Gasmesssensor (03) in der Messeinrichtung (01) hingeleitet und gemessen werden können,
**dadurch gekennzeichnet,**
**dass** vor dem Gasmesssensor (03) zumindest eine Schaumschutzeinrichtung (08, 09) vorgesehen ist, mit der der Durchtritt von im Fermentationsgefäß gebildetem Schaum zum Gasmesssensor (03) verhindert werden kann.

2. Messeinrichtung nach Anspruch 1,
**dadurch gekennzeichnet,**
**dass** die Schaumschutzeinrichtung in der Art einer passiv wirkenden Schaumschutzeinrichtung (08) mit einer Schaumbarriere (12) ausgebildet ist.

3. Messeinrichtung nach Anspruch 2,
**dadurch gekennzeichnet,**
**dass** die Schaumbarriere (12) von einem gasdurchlässigen Gewebe gebildet wird.

4. Messeinrichtung nach Anspruch 3,
**dadurch gekennzeichnet,**
**dass** das Gewebe der Schaumbarriere (12) aus einem hydrophoben Material, insbesondere aus PET oder PTFE, hergestellt ist.

5. Messeinrichtung nach Anspruch 3 oder 4,
**dadurch gekennzeichnet,**
**dass** das Gewebe der Schaumbarriere (12) eine 1:2 Köper-Bindung aufweist.

6. Messeinrichtung nach einem der Ansprüche 3 bis 5,
**dadurch gekennzeichnet,**
**dass** das Gewebe der Schaumbarriere (12) eine Maschenweite von 10 bis 40 µm, insbesondere 27 µm, aufweist.

7. Messeinrichtung nach einem der Ansprüche 3 bis 6,
**dadurch gekennzeichnet,**
**dass** das Gewebe der Schaumbarriere (12) eine Siebfeinheit von 100/cm bis 200/cm, insbesondere 146/cm, aufweist.

8. Messeinrichtung nach einem der Ansprüche 3 bis 7,
**dadurch gekennzeichnet,**
**dass** das Gewebe der Schaumbarriere (12) einen Drahtdurchmesser von 30 µm bis 50 µm, insbesondere 42 µm, aufweist.

9. Messeinrichtung nach einem der Ansprüche 3 bis 8,
**dadurch gekennzeichnet,**
**dass** das Gewebe der Schaumbarriere (12) eine offene Fläche von 10 % bis 20 %, insbesondere 15,5 %, aufweist.

10. Messeinrichtung nach einem der Ansprüche 3 bis 9,
**dadurch gekennzeichnet,**
**dass** das Gewebe der Schaumbarriere (12) ein Flächengewicht von 40 g/m² bis 50 g/m², insbesondere 45 g/m², aufweist.

11. Messeinrichtung nach einem der Ansprüche 1 bis 10,
**dadurch gekennzeichnet,**
**dass** die Schaumschutzeinrichtung in der Art einer aktiv wirkenden Schaumschutzeinrichtung (09) mit einem Schaumsensor (10) und einer Absperreinrichtung (11) ausgebildet ist, wobei die Absperreinrichtung (11) selbsttätig verschlossen wird, sobald der Schaumsensor (10) das Vorhandensein von Schaum detektiert.

12. Messeinrichtung nach Anspruch 11,
**dadurch gekennzeichnet,**
**dass** die aktiv wirkende Schaumschutzeinrichtung (09) der passiv wirkenden Schaumschutzeinrichtung (08) vorgeordnet ist.

13. Messeinrichtung Anspruch 11 oder 12,
**dadurch gekennzeichnet,**
**dass** die Absperreinrichtung (11) in der Art eines Ventils, insbesondere in der Art eines Membranmagnetventils oder eines motorisch angetriebenen Kugelventils, ausgebildet ist.

14. Messeinrichtung nach einem der Ansprüche 11 bis 13,
**dadurch gekennzeichnet,**
**dass** der Schaumsensor (10) in der Art eines kapazitiven und/oder conduktiven und/oder optischen Sensors ausgebildet ist.

15. Messeinrichtung nach einem der Ansprüche 11 bis 14,
**dadurch gekennzeichnet,**
**dass** die Kontaktfläche des Schaumsensors (10), an der der Schaum zur Anlage kommen kann, aus einem schmutz- und wasserabweisenden Material, insbesondere aus PET oder PTFE, hergestellt ist.

16. Messeinrichtung nach einem der Ansprüche 11 bis 15,
**dadurch gekennzeichnet,**
**dass** der Schaumsensor (10) in oder an einem Detektionsraum angeordnet ist, wobei der Querschnitt des Detektionsraums so groß ist, dass aufgrund des Abstandes zwischen Schaumsensor (10) und gegenüberliegender Innenwandung des Detektionsraums eine dauerhafte Schaumanhaftung ausgeschlossen ist.

17. Messeinrichtung nach einem der Ansprüche 1 bis 16,
**dadurch gekennzeichnet,**
**dass** eine Überdruckschutzeinrichtung (13) vorgesehen ist, wobei die Überdruckschutzeinrichtung (13) bei Überschreiten einer Überdruckgrenze Gas und/oder Schaum ablässt.

18. Messeinrichtung nach Anspruch 17,
**dadurch gekennzeichnet,**
**dass** die Überdruckgrenze der Überdruckschutzeinrichtung (13) kleiner als der Druck ist, der durch die schaumbenetzte Schaumbarriere (12) abgedichtet werden kann.

19. Messeinrichtung nach Anspruch 17 oder 18,
**dadurch gekennzeichnet,**
**dass** die Überdruckschutzeinrichtung (13) einen mit Flüssigkeit (14) gefüllten Siphon (15) umfasst.

20. Messeinrichtung nach Anspruch 19,
**dadurch gekennzeichnet,**
**dass** der Füllstand der Flüssigkeit (14) im Siphon (15) mit einer Füllstandsüberwachungseinrichtung (17) überwacht werden kann.

21. Messeinrichtung nach Anspruch 19 oder 20,
**dadurch gekennzeichnet,**
**dass** am äußeren Ende des Siphons (15) ein Speichervolumen (16) vorgesehen ist, in dem beim Gas- und/oder Schaumaustritt durch den Siphon (15) die Flüssigkeit (14) aufgenommen werden kann.

22. Messeinrichtung nach einem der Ansprüche 1 bis 21,
**dadurch gekennzeichnet,**
**dass** die Messeinrichtung (01) zumindest bereichsweise mit zumindest einem Heizelement (18) beheizt werden kann.

23. Messeinrichtung nach einem der Ansprüche 1 bis 22,
**dadurch gekennzeichnet,**
**dass** die Messeinrichtung (01) zumindest bereichsweise mit einer Wärmeisolierung (19) ausgestattet ist.

24. Messeinrichtung nach einem der Ansprüche 1 bis 23,
**dadurch gekennzeichnet,**
**dass** der Gasmesssensor (03) in der Art eines Gasdurchflusssensors, insbesondere in der Art eines Massendurchflusssensors, ausgebildet ist.

25. Messeinrichtung nach einem der Ansprüche 1 bis 24,
**dadurch gekennzeichnet,**
**dass** parallel zum Gasmesssensor (03) zumindest ein Bypass (20) mit definierter Durchflusskennlinie vorgesehen ist.

26. Messeinrichtung nach einem der Ansprüche 1 bis 25,
**dadurch gekennzeichnet,**
**dass** der Gasmesssensor (03) mit einer optischen und/oder akustischen Ausgabeeinheit (07) verbunden ist, wobei die Messwerte des Gasmesssensors (03) an der Ausgabeeinheit (07) optisch, insbesondere in der Art einer Balkenanzeige, und/oder akustisch, insbesondere in der Art eines elektronisch simulierten Gluckergeräusches, ausgegeben werden können.

27. Messeinrichtung nach einem der Ansprüche 1 bis 26,
**dadurch gekennzeichnet,**
**dass** am Gasaustritt (04) der Messeinrichtung (01) eine Schmutzbarriere, insbesondere ein gasdurchlässiges Schmutzfanggewebe (05), und/oder eine Schutzkappe (06) vorgesehen ist.

## Claims

1. A measuring device (01) for determining the activity of a fermentation process proceeding in a fermentation vessel, in particular a fermentation process, in which the measuring device (01) can be is connected to the fermentation vessel via a connecting line (02) and in which gaseous reaction products can be routed from the fermentation vessel through the connecting line (02) to a gas measurement sensor (03) in the measuring device (01) and measured, **characterized in that** at least one foaming protection device (08, 09) is provided upstream from the gas measurement sensor (03), with which the passage of foam formed in the fermentation vessel to the gas measurement sensor (03) can be prevented.

2. A measuring device according to claim 1, **characterized in that** the foaming protection device is constructed in the manner of a passively acting foaming protection device (08) with a foam barrier (12).

3. A measuring device according to claim 2, **characterized in that** the foam barrier (12) is constituted by a gas-permeable fabric.

4. A measuring device according to claim 3, **characterized in that** the fabric of the foam barrier (12) is made from a hydrophobic material, in particular from PET or PTFE.

5. A measuring device according to claim 3 or 4, **characterized in that** the fabric of the foam barrier (12) has a 1:2 twill weave.

6. A measurement device according to one of the claims 3 to 5, **characterized in that** the fabric of the foam barrier (12) has a mesh width of 10 to 40 µm, in particular 27 µm.

7. A measuring device according to one of the claims 3 to 6, **characterized in that** the fabric of the foam barrier (12) has a sieve fineness of 100/cm to 200/cm, in particular 146/cm.

8. A measurement device according to one of the claims 3 to 7, **characterized in that** the fabric of the foam barrier (12) has a filament diameter of 30 to 50 µm, in particular 42 µm.

9. A measurement device according to one of the claims 3 to 8, **characterized in that** the fabric of the foam barrier (12) has an open area of 10% to 20%, in particular 15.5%.

10. A measuring device according to one of the claims 3 to 9, **characterized in that** the fabric of the foam barrier (12) has a basis weight of 40 g/m² to 50 g/m², in particular 45 g/m².

11. A measuring device according to one of the claims 1 to 10, **characterized in that** the foaming protection device is constructed in the manner of a actively acting foaming protection device (09) with a foam sensor (10) and a shut-off device (11), where the shut-off device (11) closes automatically as soon as the foam sensor (10) detects the presence of foam.

12. A measuring device according to claim 11, **characterized in that** the actively acting foaming protection device (09) is placed before the passively acting foaming protection device (08).

13. A measuring device according to one of the claims 11 to 12, **characterized in that** the shut-off device (11) is constructed in the manner of a membrane solenoid valve or a motor-driven ball valve.

14. A measuring device according to one of the claims 11 to 13, **characterized in that** the foam sensor (10) is constructed in the manner of a capacitive and/or conductive and/or optical sensor.

15. A measuring device according to one of the claims 11 to 14, **characterized in that** the contact area of the foam sensor (10) on which the foam can come to the system is made of a material which repels dirt and water, in particular made of PET or PTFE.

16. A measuring device according to one of the claims 11 to 15, **characterized in that** the foam sensor (10) is arranged in or on a detection chamber, where the cross section of the detection chamber is so large that the spacing between the foam sensor (10) and the opposite interior wall of the detection chamber prevents a continuous adhesion of foam.

17. A measuring device according to one of the claims 1 to 16, **characterized in that** a device to protect against excess pressure (13) is provided, where the device to protect against excess pressure (13) discharges gas and/or foam when a positive pressure limit is exceeded.

18. A measuring device according to Claim 17, **characterized in that** the positive pressure limit of the device to protect against excess pressure (13) is less than the pressure which can be held by the foam barrier (12) when wetted with foam.

19. A measuring device according to claim 17 or 18, **characterized in that** the device to protect against excess pressure (13) comprises a siphon (15) filled with fluid (14).

20. A measuring device according to claim 19, **characterized in that** the fill level of the liquid (14) in the siphon (15) can be monitored with a fill level monitoring device (17).

21. A measuring device according to claim 19 or 20, **characterized in that** on the outside end of the siphon (15) a storage volume (16) is provided in which during the discharge of gas and/or foam through the siphon (15) the liquid (14) can be accommodated.

22. A measuring device according to one of the claims 1 to 21, **characterized in that** the measuring device (01) can be heated at least in sections with at least one heating element (18).

23. A measuring device according to one of the claims 1 to 22, **characterized in that** the measuring device (01) is equipped with thermal insulation (19) at least in sections.

24. A measuring device according to one of the claims 1 to 23, **characterized in that** the gas measurement sensor (03) is constructed in the manner of a gas flow sensor, in particular as a mass flow sensor.

25. A measuring device according to one of the claims 1 to 24, **characterized in that** at least one bypass (20) with a defined flow curve is provided parallel to the gas measurement sensor (03).

26. A measuring device according to one of the claims 1 to 25, **characterized in that** the gas measurement sensor (03) is connected to a visual and/our acoustic output unit (07), in which the measurements of the gas measurement sensor (03) can be output visually on the output unit (07), in particular as a kind of bar display, and/or output acoustically, in particular as a kind of electronically simulated clucking sound.

27. A measuring device according to one of the claims 1 to 26, **characterized in that** a dirt barrier, in particular a gas-permeable dirt-catching fabric(05), and/or a protective cap (06) is provided at the gas outlet (04) of the measuring device (01).

## Revendications

1. Équipement de mesure (01) pour la détermination de l'activité d'un processus de fermentation se déroulant dans un fermentateur, en particulier d'un processus de fermentation anaérobique, l'équipement de mesure (01) pouvant être relié avec le fermentateur par une conduite de raccordement (02), et les produits de réactions gazeux pouvant être conduits du fermentateur vers un capteur de gaz (03) dans l'équipement de mesure (01) et mesurés, **caractérisé en ce que**, devant le capteur de gaz (03), au moins un dispositif de protection contre l'écume (08, 09) est prévu, au moyen duquel le passage d'écume formée dans le fermentateur vers le capteur de gaz (03) peut être empêché.

2. Équipement de mesure selon la revendication 1, **caractérisé en ce que** le dispositif de protection contre l'écume est formé avec une barrière à écume (12), sous la forme d'un dispositif de protection contre l'écume (08) agissant de façon passive.

3. Équipement de mesure selon la revendication 2, **caractérisé en ce que** la barrière à écume (12) est formée par un tissu perméable aux gaz.

4. Équipement de mesure selon la revendication 3, **caractérisé en ce que** le tissu de la barrière à écume (12) est fabriqué dans un matériau hydrophobe, en particulier en PET ou en PTFE.

5. Équipement de mesure selon la revendication 3 ou 4, **caractérisé en ce que** le tissu de la barrière à écume (12) présente une armure sergée 1/2.

6. Équipement de mesure selon l'une des revendications 3 à 5, **caractérisé en ce que** le tissu de la barrière à écume (12) présente une largeur de maille de 10 à 40 µm, en particulier de 27 µm.

7. Équipement de mesure selon l'une des revendications 3 à 6, **caractérisé en ce que** le tissu de la barrière à écume (12) présente un compte de mailles de 100/cm à 200/cm, en particulier de 146/cm.

8. Équipement de mesure selon l'une des revendications 3 à 7, **caractérisé en ce que** le tissu de la barrière à écume (12) présente un diamètre de fil de 30 à 50 µm, en particulier de 42 µm.

9. Équipement de mesure selon l'une des revendications 3 à 8, **caractérisé en ce que** le tissu de la barrière à écume (12) présente une surface ouverte de 10 % à 20 %, en particulier de 15,5 %.

10. Équipement de mesure selon l'une des revendications 3 à 9, **caractérisé en ce que** le tissu de la barrière à écume (12) présente un poids surfacique de 40 g/m² à 50 g/m², en particulier de 45 g/m².

11. Équipement de mesure selon l'une des revendications 1 à 10, **caractérisé en ce que** le dispositif de protection contre l'écume est formé avec un capteur d'écume (10) et un dispositif de blocage (11), sous la forme d'un dispositif de protection contre l'écume (09) agissant de façon active, le dispositif de blocage (11) étant fermé automatiquement dès que le capteur d'écume (10) détecte la présence d'écume.

12. Équipement de mesure selon la revendication 11, **caractérisé en ce que** le dispositif de protection contre l'écume (09) agissant de façon active est disposé en amont du dispositif de protection contre l'écume (08) agissant de façon passive.

13. Équipement de mesure selon la revendication 11 ou 12, **caractérisé en ce que** le dispositif de blocage (11) est formé sous la forme d'une vanne, en particulier sous la forme d'une électrovanne à membrane ou d'un clapet à bille entraîné par moteur.

14. Équipement de mesure selon l'une des revendications 11 à 13, **caractérisé en ce que** le capteur d'écume (10) est formé sous la forme d'un capteur capacitif et/ou conductif et/ou optique.

15. Équipement de mesure selon l'une des revendications 11 à 14, **caractérisé en ce que** la surface de contact du capteur d'écume (10) à laquelle l'écume peut venir s'appliquer est fabriquée dans un matériau hydrofuge et antisalissure, en particulier en PET ou en PTFE.

16. Équipement de mesure selon l'une des revendications 11 à 15, **caractérisé en ce que** le capteur d'écume (10) est disposé dans ou sur un espace de détection, la taille de la section de l'espace de détection étant tellement importante que, en raison de la distance entre le capteur d'écume (10) et la paroi intérieure de l'espace de détection lui étant opposée, une adhérence durable de l'écume est exclue.

17. Équipement de mesure selon l'une des revendications 1 à 16, **caractérisé en ce qu'**un dispositif de protection contre les surpressions (13) est prévu, le dispositif de protection contre les surpressions (13) évacuant du gaz et/ou de l'écume lors du dépassement d'une limite de surpression.

18. Équipement de mesure selon la revendication 17, **caractérisé en ce que** la limite de surpression du dispositif de protection contre les surpressions (13) est inférieure à la pression qui peut être contenue par la barrière à écume (12) humectée d'écume.

19. Équipement de mesure selon les revendications 17 ou 18, **caractérisé en ce que** le dispositif de protection contre les surpressions (13) comprend un siphon (15) rempli avec un liquide (14).

20. Équipement de mesure selon la revendication 19, **caractérisé en ce que** le niveau du liquide (14) dans le siphon (15) peut être surveillé avec un dispositif de contrôle du niveau (17).

21. Équipement de mesure selon les revendications 19 ou 20, **caractérisé en ce que**, à l'extrémité extérieure du siphon (15), un volume tampon (16) est prévu, dans lequel le liquide (14) peut être recueilli en cas de sortie de gaz et/ou d'écume par le siphon.

22. Équipement de mesure selon l'une des revendications 1 à 21, **caractérisé en ce que** l'équipement de mesure (01) peut être chauffé au moins dans certaines zones, avec au moins un élément chauffant (18).

23. Équipement de mesure selon l'une des revendications 1 à 22, **caractérisé en ce que** l'équipement de mesure (01) est muni d'une isolation thermique (19) au moins dans certaines zones.

24. Équipement de mesure selon l'une des revendications 1 à 23, **caractérisé en ce que** le capteur de gaz (03) est formé sous la forme d'un capteur de débit de gaz, en particulier sous la forme d'un capteur de débit de masse.

25. Équipement de mesure selon l'une des revendications 1 à 24, **caractérisé en ce que**, parallèlement au capteur de gaz (03), au moins une dérivation (20) avec caractéristique de débit définie est prévue.

26. Équipement de mesure selon l'une des revendications 1 à 25, **caractérisé en ce que** le capteur de gaz (03) est relié à une unité de sortie visuelle (07) et/ou sonore, les valeurs de mesure du capteur de gaz (03) pouvant être sorties sur l'unité de sortie (07) de façon visuelle, en particulier sous la forme d'un diagramme à barres, et/ou sonore, en particulier sous la forme d'un bruit de glouglou simulé électroniquement.

27. Équipement de mesure selon l'une des revendications 1 à 26, **caractérisé en ce que**, à la sortie de gaz (04) de l'équipement de mesure (01), une barrière contre les salissures, en particulier un tissu de rétention des salissures (05) perméable aux gaz et/ou un couvercle de protection (06) est prévue.
